(19) Europäisches Patentamt
European Patent Office
Office européen des brevets

(11) EP 0 703 886 B1

(12) **EUROPEAN PATENT SPECIFICATION**

(45) Date of publication and mention of the grant of the patent:
**30.10.2002 Bulletin 2002/44**

(21) Application number: **94920006.7**

(22) Date of filing: **13.05.1994**

(51) Int Cl.[7]: **B01J 31/02**, B01J 29/06, B01J 37/08, C07C 2/00, C07C 5/52, C07C 6/12

(86) International application number:
**PCT/US94/05390**

(87) International publication number:
**WO 94/027934 (08.12.1994 Gazette 1994/27)**

# (54) PROCESS FOR MODIFYING THE SHAPE SELECTIVITY OF A ZEOLITE CATALYST AND USE OF THE MODIFIED CATALYST

VERFAHREN ZUR MODIFIKATION DER FORMSELEKTIVITÄT EINES ZEOLITHKATALYSATORS UND VERWENDUNG DES MODIFIZIERTEN KATALYSATORS

PROCEDE POUR MODIFIER LA SELECTIVITE DE FORME D'UN CATALYSEUR A LA ZEOLITE ET UTILISATION DU CATALYSEUR AINSI MODIFIE

(84) Designated Contracting States:
**BE DE DK ES FR GB IT NL SE**

(30) Priority:
**28.05.1993 US 69251**
**28.05.1993 US 69254**
**28.05.1993 US 69255**
**28.05.1993 US 69259**
**28.05.1993 US 69257**
**28.05.1993 US 69260**
**27.10.1993 US 141758**

(43) Date of publication of application:
**03.04.1996 Bulletin 1996/14**

(73) Proprietor: **EXXONMOBIL OIL CORPORATION**
**Fairfax, VA 22037 (US)**

(72) Inventors:
- **BECK, Jeffrey, Scott**
  **Princeton, NJ 08540 (US)**
- **CHANG, Clarence, Dayton**
  **Princeton, NJ 08540 (US)**
- **LUTNER, John, Dennis**
  **Hamilton Square, NJ 08690 (US)**
- **McCULLEN, Sharon, Brawner**
  **Newton, PA 18940 (US)**
- **OLSON, David, Harold**
  **Pennington, NJ 08534 (US)**
- **RODEWALD, Paul, Gerhard, Jr.**
  **Rocky Hill, NJ 08553 (US)**
- **SHIHABI, David, Said**
  **Pennington, NJ 08534 (US)**
- **VENKAT, Chaya, Rao**
  **Princeton, NJ 08540 (US)**

(74) Representative: **Hucker, Charlotte Jane et al**
**Gill Jennings & Every**
**Broadgate House,**
**7 Eldon Street**
**London EC2M 7LH (GB)**

(56) References cited:
**US-A- 4 477 583**
**US-A- 5 321 183**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 703 886 B1

## Description

The present invention relates to a process for modifying the shape selectivity of a zeolite catalyst and use of the modified catalyst in shape selective hydrocarbon conversion processes, such as toluene disproportionation.

The term "shape-selective catalysis" describes unexpected catalytic selectivities in zeolites. The principles behind shape selective catalysis have been reviewed extensively, e.g., by N.Y. Chen, W.E. Garwood and F.G. Dwyer, "Shape Selective Catalysis in Industrial Applications," 36, Marcel Dekker, Inc. (1989). Within a zeolite pore, hydrocarbon conversion reactions such as isomerization, disproportionation, alkylation and transalkylation of aromatics are governed by constraints imposed by the channel size. Reactant selectivity occurs when a fraction of the feedstock is too large to enter the zeolite pores to react; while product selectivity occurs when some of the products cannot leave the zeolite channels. Product distributions can also be altered by transition state selectivity in which certain reactions cannot occur because the reaction transition state is too large to form within the zeolite pores or cages. Another type of selectivity results from configurational constraints on diffusion where the dimensions of the molecule approach that of the zeolite pore system. A small change in the dimensions of the molecule or the zeolite pore can result in large diffusion changes leading to different product distributions. This type of shape selective catalysis is demonstrated, for example, in selective alkyl-substituted benzene disproportionation to para-dialkyl-substituted benzene.

A representative para-dialkyl-substituted benzene is para-xylene. The production of para-xylene is typically performed by methylation of toluene or by toluene disproportionation over a catalyst under conversion conditions. Examples include the reaction of toluene with methanol, as described by Chen et al., J. Amer. Chem. Soc. 101, 6783 (1979), and toluene disproportionation, as described by Pines in "The Chemistry of Catalytic Hydrocarbon Conversions", Academic Press, N.Y., 1981, p. 72. Such methods typically result in the production of a mixture of the three xylene isomers, i.e., para-xylene, ortho-xylene, and meta-xylene. Depending upon the degree of selectivity of the catalyst for para-xylene (para-selectivity) and the reaction conditions, different percentages of para-xylene are obtained. The yield, i.e., the amount of xylene produced as a proportion of the feedstock, is also affected by the catalyst and the reaction conditions.

The equilibrium reaction for the conversion of toluene to xylene and benzene proceeds as follows:

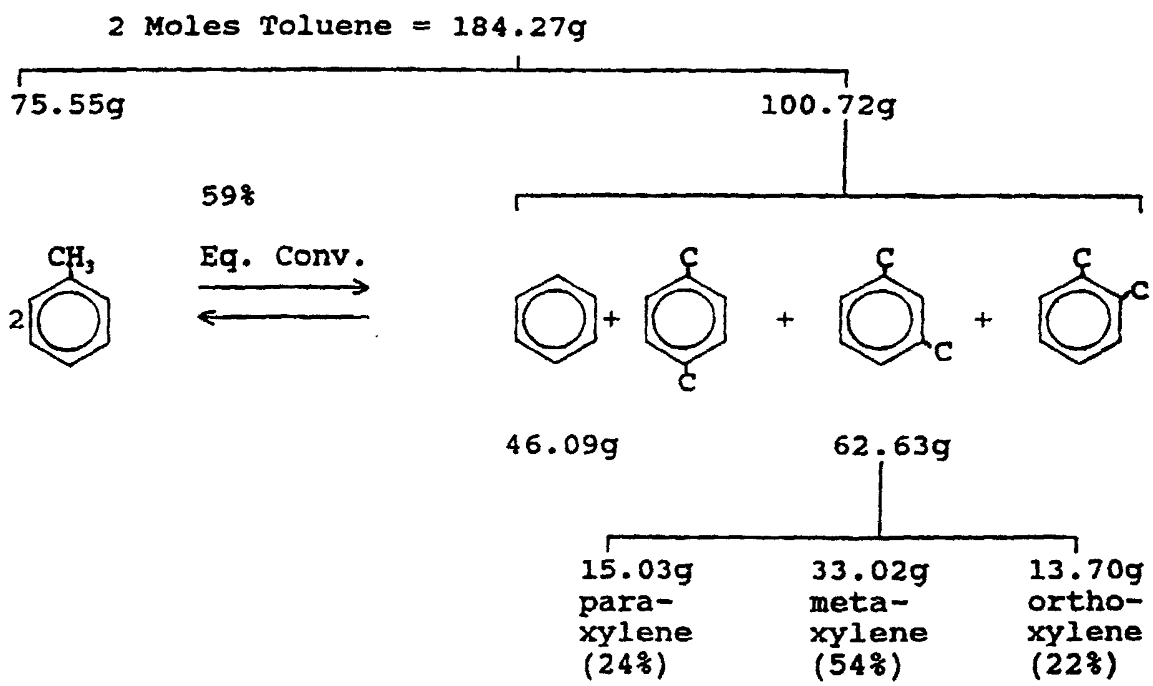


$$\text{p-Xylene Yield} = 100 \times \frac{15.03}{184.27} = 8.2\%$$

$$\text{p-Xylene Selectivity} = 100 \times \frac{15.03}{62.63} = 24\%$$

Various methods are known in the art for increasing the para-selectivity of zeolite catalysts. One such method

is to modify the catalyst by treatment with a "selectivating agent". For example, U.S. Patents 5,173,461, 4,950,835, 4,927,979, 4,465,886, 4,477,583, 4,379,761, 4,145,315, 4,127,616, 4,100,215, 4,090,981, 4,060,568 and 3,698,157 disclose specific methods for contacting a catalyst with a selectivating agent containing silicon ("silicon compound"). U.S. Patent No. 4,548,914 describes another modification method involving impregnating catalysts with oxides that are difficult to reduce, such as those of magnesium, calcium, and/or phosphorus, followed by treatment with water vapor to improve para-selectivity.

European Patent No. 296,582 describes the modification of aluminosilicate catalysts by impregnating such catalysts with phosphorus-containing compounds and further modifying these catalysts by incorporating metals such as manganese, cobalt, silicon and Group IIA elements. The patent also describes the modification of zeolites with silicon compounds.

Traditionally, ex situ pre-selectivation of a zeolite, that is selectivation outside the reaction vessel and prior to the desired reaction, has involved a single application of the modifying compound. It may be noted, however, that the suggestion of multiple treatments was made in U.S. Patent No. 4,283,306 to Herkes. The Herkes patent discloses the promotion of a crystalline silica catalyst by application of an amorphous silica such as ethylorthosilicate. The Herkes patent contrasts the performance of the catalyst treated once with an ethylorthosilicate solution followed by calcination with the performance of the catalyst treated twice with ethylorthosilicate and calcined after each treatment. The Herkes disclosure shows that the twice-treated catalyst is less active and less selective than the once-treated catalyst as measured by methylation of toluene with methanol, suggesting that multiple ex situ selectivation confers no benefit and in fact reduces a catalyst's efficacy in shape-selective reactions.

There has been no suggestion, however, that the selectivation of aluminosilicate zeolites by the multiple ex situ impregnation of the zeolites with silicon compounds, followed by calcination after each impregnation would improve the selectivity and activity of the catalysts. It has now been found that a multiple impregnation scheme provides unexpectedly better results in shape-critical hydrocarbon conversions, such as toluene disproportionation, than single silicon impregnation pretreatment schemes.

It has also now been found that a multiple impregnation scheme provides unexpectedly more efficient deposition of the silicon compound on the catalyst than single silicon impregnation schemes.

Steaming has also been used in the preparation of zeolite catalysts to modify their activity or improve their stability. For example, U.S. Patent No. 4,559,314 describes steaming a zeolite/binder composite at 200-500°C for at least an hour to enhance activity as measured by its alpha value. U.S. Patent No. 4,522,929 describes presteaming a fresh zeolite catalyst so that the alpha activity first rises then falls to the level of the fresh unsteamed catalyst, producing a stable catalyst which may be used in xylene isomerization. U.S. Patent No. 4,443,554 describes steaming inactive zeolites (Na ZSM-5) to increase alpha activity. U.S. Patent No. 4,487,843 describes contacting a zeolite with steam prior to loading with a Group IIIB metal.

It has also now been found that a multiple silicon impregnation scheme for zeolite catalyst selectivation followed by steam treatment under specific conditions produces additional unexpectedly improved results than the multiple impregnation treatment alone.

Accordingly, the invention resides in a process for modifying the shape selectivity of an aluminosilicate zeolite catalyst having a silica to alumina molar ratio less than 500 and comprising exposing the catalyst to at least two ex situ selectivation sequences, wherein each ex situ selectivation sequence includes the steps of contacting the catalyst with a selectivating agent in a carrier and subsequently calcining the catalyst, wherein the selectivating agent is an organosilicon compound.

The invention also resides in the use of the modified catalyst in shape selective hydrocarbon conversions, in particular para-selective alkylaromatic conversions, such as toluene disproportionation and ethylbenzene disproportionation.

The zeolites useful herein have a Constraint Index from 1 to 12 and a silica to alumina molar ratio less than 500 and normally greater than 12, preferably 20 to 100 and most preferably 20 to 60. Suitable zeolites include ZSM-5, ZSM-11, ZSM-12, ZSM-22, ZSM-23, ZSM-35, ZSM-48, ZSM-50, and ZSM-57. Such zeolites are described, for example, in U.S. Patent Nos. 3,702,886 and Re. No. 29,949, 3,709,979, 3,832,449, 4,046,859, 4,556,447, 4,076,842, 4,016,245, 4,229,424, 4,397,827, 4,640,849, 4,046,685, 3,308,069 and Re. 28,341. Constraint Index and the manner by which it is determined is described in, for example, U.S. Patent No. 4,016,218.

In addition to one or more of the above-mentioned zeolites, the catalyst modified by the process of the invention may comprise a binder conventionally employed to impart physical strength and attrition resistance to the catalyst. The binder is preferably free of alumina and most preferably contains silica. The binder may comprise 2 to 70, preferably 20 to 50, wt% of the catalyst.

According to the present invention, the zeolite, either incorporated with a binder or in unbound form, is contacted at least twice, preferably between two and six times, with a selectivating agent, preferably in the form of an organosilicon compound. The selectivating agent is present in a carrier, which can be an aqueous or an organic carrier, and after each contacting step the catalyst is calcined to remove the carrier and any organic component of the selectivating agent. In each phase of the selectivation treatment, the selectivating agent is deposited on the external surface of the catalyst by any suitable method. For example, the selectivating agent may be dissolved in an organic carrier, mixed with the catalyst, and then dried by evaporation or vacuum distillation. This method is termed "impregnation". The molecular sieve may be contacted with the silicon compound at a molecular sieve/silicon compound weight ratio of 100/1 to 1/100.

Preferably, the kinetic diameter of the selectivating agent is larger than the zeolite pore diameter, in order to avoid entry of the selectivating agent into the pore and any concomitant reduction in the internal activity of the catalyst.

The selectivating agent employed may be in the form of a solution, an emulsion, a liquid or a gas under the conditions of contact with a zeolite. The deposited selectivating agent extensively covers, and resides substantially exclusively on, the external surface of the molecular sieve. Examples of methods of depositing silicon on the surface of the zeolite are found in U.S. Patent Nos. 4,090,981, 4,127,616, 4,465,886 and 4,477,583 to Rodewald. Further examples of the deposition of a silicon compound on zeolite surfaces are described in H. Nakajima, M. Koya, H. Ishida, and M. Kohno, Sekiyu Gakkaishi, 35(2) (1992), and in U.S. Patent No. 4,950,835 to Wang et al. In addition, the silicon selectivating agent can be combined with the zeolite during catalyst particle formation, for example, by adding the selectivating agent/carrier combination together with the zeolite and optionally a binder, to a muller which is then used to form the mixture into an extrudable mass from which catalyst particles can be extruded.

Useful selectivating agents include siloxanes which have repeating units characterized by the general formula:

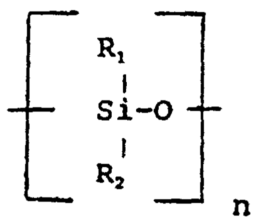

where $R_1$ is hydrogen, halogen, hydroxyl, alkyl, halogenated alkyl, aryl, halogenated aryl, aralkyl, halogenated aralkyl, alkaryl or halogenated alkaryl. The hydrocarbon substituents generally contain from 1 to 10 carbon atoms, preferably methyl or ethyl groups. $R_2$ is independently selected from the same group as $R_1$, and n is an integer of at least 2 and generally in the range of 3 to 1000. The molecular weight of the silicone compound employed is generally between 80 and 20,000 and preferably within the approximate range of 150 to 10,000. Representative silicone compounds include dimethyl silicone, diethyl silicone, phenylmethyl silicone, methylhydrogen silicone, ethylhydrogen silicone, phenylhydrogen silicone, methylethyl silicone, phenylethyl silicone, diphenyl silicone, methyltrifluoropropyl silicone, ethyltrifluoropropyl silicone, polydimethyl silicone, tetrachlorophenylmethyl silicone, tetrachlorophenylethyl silicone, tetrachlorophenylhydrogen silicone, tetrachlorophenylphenyl silicone, methylvinyl silicone and ethylvinyl silicone. The silicone compound need not be linear, but may be cyclic, for example, hexamethyl cyclotrisiloxane, octamethyl cyclotetrasiloxane, hexaphenyl cyclotrisiloxane and octaphenyl cyclotetrasiloxane. Mixtures of these compounds may also be used, as may silicones with other functional groups.

Other silicon compounds, including silanes and alkoxy silanes, such as tetramethoxy silane, may also be utilized. These useful silicon-containing selectivating agents include silanes characterizable by the general formula:

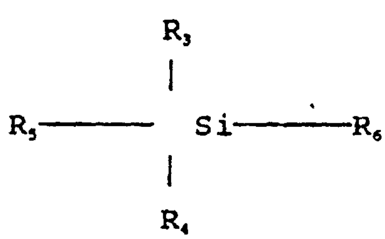

where $R_1$, $R_2$, $R_3$ and $R_4$ are independently selected from the group consisting of hydrogen, hydroxyl, halogen, alkyl, halogenated alkyl, alkoxy, alkylamino, aryl, halogenated aryl, aralkyl, halogenated aralkyl, alkaryl, and halogenated alkaryl groups. Alkylaminosilanes, because of their amphiphilic character, may be dissolved, or at least emulsified, in aqueous carriers.

Preferred-silicon-containing selectivating agents include dimethylphenylmethyl polysiloxane (e.g., Dow-550) and phenylmethyl polysiloxane (e.g., Dow-710). Dow-550 and Dow-710 are available from Dow Chemical Co., Midland, Mi.

In the aqueous selectivation systems of the invention, the most preferred silicon selectivating agents include an n-propylamine silane, available as Hydrosil 2627 from Hüls America.

In one embodiment of the present invention, the carrier for the selectivating agent is an organic medium. Examples of suitable organic carriers are linear, branched, and cyclic alkanes having 5 or more carbon atoms, preferably 7 or more carbon atoms. In the process of the present invention it is preferred that the organic carrier has a boiling point greater than about 70°C. Optionally, mixtures of low volatility organic compounds, such as hydrocracker recycle oil, may be employed as carriers. The most preferred low volatility hydrocarbon carriers are decane and dodecane.

Alternatively, the carrier for the selectivating agent is an aqueous medium which in the case of certain of the silicon compounds described above, for example Hydrosil 2627, can be used without an emulsifier. However, in most cases, the aqueous,medium will require the addition of a surfactant to form an emulsion between the silicon selectivating agent and the aqueous carrier. Conveniently, the emulsions contain 0.01 to 5 wt%, preferably 0.05 to 2 wt%, surfactant and 2.5 to 50 wt%, preferably 5 to 35 wt%, of the silicon selectivating agent.

Although aqueous silicone emulsions are commercially available, they may contain significant amounts of added organic materials, such as toluene, for improving stability. The presence of such additional organics increases the complexity, hazards, and cost of zeolite selectivation. Such emulsions are therefore useful, but not preferred, in accordance with the catalyst modification methods of the present invention. With proper formulation, the inventors have succeeded in producing stable emulsions containing a silicone oil, an aqueous component, and a surfactant, while substantially devoid of other components.

The preferred, stable aqueous emulsions of silicone oil are generated by mixing the oil and an aqueous component in the presence of a surfactant or surfactant mixture. Surfactants useful for the present invention include any of a large variety of surfactants, including ionic and non-ionic surfactants. Preferred surfactants include non-nitrogenous non-ionic surfactants such as alcohol, alkylphenol, and polyalkoxyalkanol derivatives, glycerol esters, polyoxyethylene esters, anhydrosorbitol esters, ethoxylated anhydrosorbitol esters, natural fats, oils, waxes and ethoxylated esters thereof, glycol esters, polyalkylene oxide block co-polymer surfactants, poly(oxyethylene-co-oxypropylene) non-ionic surfactants, and mixtures thereof. More preferred surfactants include surfactants having the formula $\alpha$-[4-(1,1,3,3-tetramethylbutyl)phenyl]-$\omega$-hydroxypoly(oxy-1,2-ethanediyl) (Octoxynols), most preferably octoxynol-9. Such preferred surfactants include the TRITON® X series, such as TRITON® X-100 and TRITON® X-305, available from Rohm & Haas Co., Philadelphia, PA, and the Igepal CA series from GAF Corp., New York, NY.

The aqueous component suitable for use in an emulsion of the invention will substantially constitute the continuous phase of the emulsion. The aqueous component is preferably water. Most preferably, the water is distilled prior to use in the emulsions. Alternatively, the aqueous component may include water and a compound selected from the group including inorganic salts, alcohols having between 1 and 18 carbons, glycols, ethers, neutral or charged sulfoxides, neutral or charged amines, aldehydes, ketones, thiophenes, furans, pyrroles, and mixtures thereof.

It has been found that a multiple selectivation scheme provides unexpectedly increased efficiency of deposition of the silicon compound on the surface of the catalyst. This increased efficiency allows for the use of relatively small quantities of the silicon compound as well as relatively small quantities of the carrier.

Following each deposition of the silicon compound, the catalyst is calcined to decompose the molecular or polymeric species to a solid state species. The catalyst may be calcined at a rate of 0.2°C/minute to 5°C/minute to a temperature greater than 250°C, but below a temperature at which the crystallinity of the zeolite is adversely affected. Generally, such temperature will be below 600°C. Preferably the temperature of calcination is within the approximate range of 350°C to 550°C. The product is maintained at the calcination temperature usually for 1 to 24 hours, preferably for between 2 and 6 hours.

The catalyst may be calcined in an atmosphere of $N_2$, an oxygen-containing atmosphere, preferably air, an atmosphere of $N_2$ followed by an oxygen-containing atmosphere, or an atmosphere containing a mixture of $N_2$ and air. Calcination should be performed in an atmosphere substantially free of water vapor, to avoid undesirable uncontrolled steaming of the silicon coated catalyst. The catalyst may be calcined once or more than once after each silicon deposition. The various calcinations in any impregnation sequence need not be identical, but may vary with respect to the temperature, the rate of temperature rise, the atmosphere and the duration of calcination.

After the selectivation sequence, the catalyst may be subjected to steam treatment at a temperature of 100°C to 600°C, preferably from 175°C to 325°C; with from 1% to 100% steam, preferably from 50% to 100% steam; at a pressure of 0.01 psia to 50 psia; for two to twelve hours, preferably from three to six hours.

In addition, the ex situ selectivated catalyst of the invention may be subjected to one or more further selectivation treatments in situ in the reactor in which the catalyst is used to effect catalytic conversion, such as the selective conversion of an alkylenzene to a para-dialkylbenzene. This in situ selectivation is referred to herein as trim-selectivation.

In one embodiment, trim selectivation is effected by feeding a second selectivating agent and hydrogen simultaneously with the alkylbenzene feedstock at reaction conditions until the desired p-dialkylbenzene selectivity, e.g., 90%, is attained, whereupon the co-feed of selectivating agent is discontinued. Reaction conditions for this in situ

trim-selectivation step generally include a temperature of 350°C to 650°C and a pressure of 100 to 34500 kPa (atmospheric to 5000 psig). The reaction stream is fed to the system at a rate of 0.1 WHSV to 20 WHSV, whereas hydrogen is fed at a hydrogen to hydrocarbon molar ratio of 0.1 to 20.

The second selectivating agent for trim-selectivation may comprise a silicon compound as discussed in greater detail above. For example, organic silicon compounds such as phenylmethyl silicone, dimethyl silicone, and mixtures thereof are suitable. According to one embodiment, a silicone containing phenylmethylsilicone and dimethylsilicone groups in a ratio of about 1:1 is co-fed to the system, while the other components, e.g., alkylbenzene and hydrogen, are fed in the amounts set forth above. The silicone is preferably fed in an amount of 0.001 wt.% to 10 wt.% of the alkylbenzene. Depending upon the percentage of selectivating agent used, the trim-selectivation will last for at least one hour, preferably 1 to 48 hours, most preferably less than 24 hrs.

In this scheme the silicon compound will decompose to deposit additional silica to on the catalyst. During the selectivation procedure the para-selectivity of the catalyst will be observed to increase further. The silicon containing polymer or molecular species may be dissolved in toluene or other appropriate aromatic or hydrocarbon carrier.

Alternatively, the ex situ selectivated catalyst, prior to contacting with the alkylbenzene feed, may be subjected to trim-selectivation with a thermally decomposable organic compound at an elevated temperature in excess of the decomposition temperature of said compound but below the temperature at which crystallinity of the zeolite is adversely affected. Generally, this temperature will be less than about 650°C. In this embodiment, the trim selectivation will generate coke on the surface of the catalyst.

Organic materials, thermally decomposable under the above temperature conditions to provide coke trimming, encompass a wide variety of compounds including by way of example, hydrocarbons, such as paraffinic, cycloparaffinic, olefinic, cycloolefinic and aromatic; oxygen-containing organic compounds such as alcohols, aldehydes, ethers, ketones and phenols; heterocyclics such as furans, thiophenes, pyrroles and pyridines. Usually, it is contemplated that a thermally decomposable hydrocarbon, such as an alkyl-substituted aromatic, will be the source of coke, most preferably the alkylbenzene being converted by the catalyst of the invention. In the latter case, the alkylbenzene is initially brought into contact with the catalyst under conditions of temperature and hydrogen concentration amenable to rapid coke formation. Typically, coke trimming is conducted at conditions more severe than the operating parameters used during the main time span of the catalytic cycle. When the desired coke deposition has been effected, the alkyl-benzene feed is continued in contact with the coke-containing catalyst under conditions of temperature and hydrogen concentration conducive to the desired conversion, with a greatly reduced coking rate.

While not wishing to be bound by theory, it is believed that the advantages of the present invention are in part obtained by rendering acid sites on the external surfaces of the catalyst substantially inaccessible to reactants, while increasing catalyst tortuosity. In the case of the selective conversion of alkylbenzene to p-dialkylbenzenes, acid sites existing on the external surface of the catalyst are believed to isomerize the solution-phase p-dialkylbenzene back to an equilibrium level with the other dialkylbenzene isomers, thereby reducing the amount of para-selectivity. By reducing the availability of these acid sites to the solution-phase p-dialkylbenzene, the relatively high proportion of the para isomer can be maintained. It is believed that the selectivating agents of the present invention block or otherwise render these external acid sites unavailable to the p-dialkylbenzene by chemically modifying said sites.

In general, catalytic conversions over the modified zeolite catalyst of the invention are conducted at a temperature of 100°C to 760°C, a pressure of 10 to 20,000 kPa (0.1 atmosphere to 200 atmospheres), a weight hourly space velocity of 0.08 to 2000, and a hydrogen/organic, e.g., hydrocarbon compound, mole ratio of 0 to 100.

Zeolites modified in accordance with the invention are generally useful as catalysts in shape selective hydrocarbon conversion processes, particularly shape selective disproportionation of alkyl-substituted benzenes to yield dialkyl-substituted benzenes. The modified zeolite catalysts of the invention are advantageously used in the conversion of alkylbenzene compounds to provide dialkylbenzene products which are highly enriched in the para-dialkylbenzene isomer. Conversion reactions of this type include transalkylation and disproportionation of alkylbenzenes. Alkylations of aromatics in which the catalysts of the invention can be used are described, for example, in U.S. Patent Nos. 3,755,483, 4,086,287, 4,117,024 and 4,117,026.

The modified catalysts of the present invention have been found to be particularly useful in the selective production of para-dialkyl-substituted benzenes containing alkyl groups of 1 to 4 carbon atoms, such as para-xylene. Such processes are typified by the disproportionation, in the presence of the modified catalyst, of a hydrocarbon precursor, typically a monoalkyl-substituted benzene having 1 to 4 carbon atoms in the alkyl substituent.

Disproportionation of Alkylbenzenes

The catalyst of the present invention is described in detail below in relation to its preferred use in the disproportionation of alkyl-substituted benzenes, such as toluene and ethylbenzene. Normally a single pass conversion of an alkylbenzene stream results in a product stream which includes dialkylbenzenes having alkyl groups at all locations, i.e., o-, m-, and p-dialkylbenzenes. A catalyst treated in the manner described herein exhibits a desirable decreased

ortho-dialkylbenzene sorption rate parameter and yields a significantly para-selected product from alkylbenzene disproportionation. For example, diffusion rate constants in toluene disproportionation have been discussed by D.H. Olson and W.O. Haag, "Structure-Selectivity Relationship in Xylene Isomerization and Selective Toluene Disproportionation", Catalytic Materials: Relationship Between Structure and Reactivity, ACS Symposium Ser. No. 248 (1984).

In toluene disproportionation, toluene diffuses into the zeolite with a diffusivity $D_T$. The toluene undergoes disproportionation to p-, m-, and o-xylene and benzene at a total rate constant $k_D$. For high selectivity and catalyst efficiency it is desirable to have:

$$k_D << \frac{D_T}{r^2}.$$

The degree of para-selectivity depends on the activity and the diffusion characteristics of the catalyst. The primary product will be rich in the para isomer if initially produced m- and o-xylene diffuse out of the zeolite crystal at a rate ($D_{m,o}/r^2$) that is lower than that of their conversion to p-xylene ($k_I$), as well as lower than that of the p-xylene diffusion ($D_p/r^2$) out of the catalyst, where:

$D_m$ = diffusion of m-xylene;
$D_o$ = diffusion of o-xylene;
$D_p$ = diffusion of p-xylene;
r = length of diffusion path (crystal size);
$k_I$ = rate of interconversion via isomerization of xylene isomers yielding secondary xylene product m-xylene and o-xylene.

It is desirable to increase the para-selectivity of the catalyst. Practically, this involves decreasing the o-and m-xylene diffusivities such that

$$k_I > \frac{D_{m,o}}{r^2}.$$

In such a case the rate of conversion of m- and o-xylenes to p-xylene exceeds the diffusivities of the m- and o-xylenes. As a result, the proportion of the xylene yield that is p-xylene will be increased. Those skilled in the art will appreciate that similar considerations apply to the diffusivities of other alkylbenzenes.

The catalyst of the invention achieves very high para-dialkylbenzene selectivity, normally greater than 80%, and preferably greater than 90%, in the disproportionation of alkylbenzenes at single pass conversion levels of at least 10%, preferably at least 15-25%. As used herein, the term "para-dialkylbenzene selectivity" means the proportion of p-dialkylbenzene, indicated as a percentage, among all of the dialkylbenzene products, i.e., p-dialkylbenzene, o-dialkylbenzene, and m-dialkylbenzene. Those skilled in the art will appreciate that the relative proximity of the boiling points of these isomers necessitates relatively expensive separation processes for the isolation of p-dialkylbenzene. On the other hand, p-dialkylbenzenes are more readily separated from other components in the product stream such as benzene, monoalkylbenzenes and other alkyl-substituted benzenes.

The alkylbenzene feedstock preferably includes 50% to 100% alkylbenzene, more preferably at least 80% alkylbenzene. Other compounds such as benzene and other alkyl-substituted benzenes may also be present in a toluene feedstock.

The alkylbenzene feedstock may also be dried, if desired, in a manner which will minimize moisture entering the reaction zone. Numerous methods known in the art are suitable for drying the alkylbenzene charge for the process of the invention. These methods include percolation through any suitable desiccant, for example, silica gel, activated alumina, molecular sieves or other suitable substances, or the use of liquid charge dryers.

When used in the disproportionation of alkylbenzenes, the zeolite selectivated by the process of the invention preferably has a crystal size greater than 0.1 micron, more preferably greater than 0.2 micron. The accurate measurement of crystal size of zeolite materials is frequently very difficult. Microscopy methods, such SEM and TEM, are often used, but these methods require measurements on a large number of crystals and for each crystal measured, values may be required in up to three dimensions. For ZSM-5 materials described in the examples below, estimates were made of the effective average crystal size by measuring the rate of sorption of 2,2-dimethylbutane at 90°C and 60 torr hydrocarbon pressure. The crystal size is computed by applying the diffusion equation given by J. Crank, "The Mathematics of Diffusion", Oxford at the Clarendon Press, 1957, pp 52-56, for the rate of sorbate uptake by a solid whose diffusion properties can be approximated by a plane sheet model. In addition, the diffusion constant of 2,2-dimethylbutane, D, under these conditions, is taken to be $1.5x10^{-14}$ $cm^2$/sec. The relation between crystal size measured in microns, d, and diffusion time measured in minutes, $t_{0.3}$, the time required for the uptake of 30% of capacity of hydrocarbon, is:

$$d = 0.0704 \times t_{0.3}^{1/2}.$$

In the present case these measurements have been made on a computer controlled, thermogravimetric electrobalance, but there are numerous ways one skilled in the art could obtain the data. The larger crystal material used herein has a sorption time, $t_{0.3}$, of 497 minutes, which gives a calculated crystal size of 1.6 microns. The smaller crystal material has a sorption time of 7.8 minutes, and a calculated crystal size of 0.20 micron.

The zeolite selectivated for use for in the disproportionation of alkylbenzenes preferably has a final alpha value of at least 50 and preferably at least 100. Alpha value gives an approximate indication of the catalytic cracking activity of a catalyst compared to a standard amorphous silica-alumina cracking catalyst. The alpha test is described in U.S. Patent 3,354,078 and in The Journal of Catalysis, Vol. 4, pp. 522-529 (August 1965); Vol. 6, p. 278 (1966); and Vol. 61, p. 395 (1980). It is noted that intrinsic rate constants for many acid-catalyzed reactions are proportional to the alpha value for a particular crystalline silicate catalyst (see "The Active Site of Acidic Aluminosilicate Catalysts," Nature, Vol. 309, No. 5959, pp. 589-591, 14 June 1984).

Operating conditions employed in the alkylbenzene disproportionation process of the present invention suitably incude a reactor inlet temperature of 200°C to 600°C, preferably 350°C to 540°C; a pressure of 100 to 34500 kPa (atmospheric to 5000 psia), preferably 700 to 7000 kPa (100 to 1000 psia); a WHSV of 0.1 to 20, preferably 2 to 10; and a $H_2$/HC mole ratio of 0.05 to 20, preferably 0.5 to 6. This process may be conducted in either batch or fluid bed operation, with the attendant benefits of either operation readily obtainable. The effluent may be separated and distilled to remove the desired product, i.e., the para isomer, as well as other by-products. Alternatively, the appropriate fraction may be subjected to further separation, as in the case of xylenes, subjected to crystallization or the PAREX process to yield p-xylene.

The catalyst may be further modified in order to reduce the amount of undesirable by-products, such as, in the case of xylenes, ethylbenzene. The state of the art is such that the reactor effluent from standard toluene disproportionation typically contains about 0.5% ethylbenzene by-product. Upon distillation of the reaction products, the level of ethylbenzene in the $C_8$ fraction often increases to between 3% and 4%. This level of ethylbenzene is unacceptable for polymer grade p-xylene since ethylbenzene in the p-xylene, if not removed, degrades the quality of fibers ultimately produced from the p-xylene product. Consequently, ethylbenzene content of the p-xylene product must be kept low. The specification for the allowable amount of ethylbenzene in the p-xylene product has been determined by the industry to be less than 0.3%. Ethylbenzene can be substantially removed by crystallization or by superfractionation processes.

In order to avoid the need for downstream ethylbenzene removal, the level of ethylbenzene by-product is advantageously reduced by incorporating a hydrogenation/dehydrogenation function within the catalyst, such as by addition of a metal compound such as platinum. While platinum is the preferred metal, other metals of Groups IB to VIII of the Periodic Table such as palladium, nickel, copper, cobalt, molybdenum, rhodium, ruthenium, silver, gold, mercury, osmium, iron, zinc, cadmium, and mixtures thereof, may be utilized. The metal may be added by cation exchange, in amounts of 0.001% to 2%, typically about 0.5% by weight of the catalyst. For example, a platinum modified catalyst can be prepared by first adding the catalyst to a solution of ammonium nitrate in order to convert the catalyst to the ammonium form. The catalyst is subsequently contacted with an aqueous solution of tetraamine platinum(II) nitrate or tetraamine platinum(II) chloride. The catalyst can then be filtered, washed with water and calcined at temperatures of 250°C to 500°C.

The following non-limiting Examples illustrate the invention in relation to the disproportionations of toluene and ethylbenzene.

In the examples, the o-xylene sorption rate parameter $D_o/r^2$ was measured at 120°C and 3.8 torr.

$D_o$ = diffusivity of o-xylene
r = crystal size

$D_o$/r2 the diffusion rate parameter is a measure of the speed of movement of o-xylene into and out of the catalyst crystals.

Also in the Examples, the atmospheric toluene disproportionation (TDP) test was performed at 482°C, 1 atmosphere, at 4% conversion. The TDP rate constant for the catalyst was obtained under these same conditions.

**EXAMPLE 1 (Comparative)**

The atmospheric TDP test to screen catalyst activity and selectivity was performed as follows using a sample of HZSM-5/$SiO_2$ (65% HZSM-5/35% $SiO_2$) with a crystal size of 1.6 microns. The untreated sample was reacted with toluene at atmospheric pressure at 482°C, and the toluene conversion was varied by adjusting the toluene WHSV. The para-selectivity of the untreated catalyst was 37% at 4% toluene conversion, with a TDP rate constant of 167. The o-xylene diffusivity of the untreated catalyst was $4.7 \times 10^{-6}$, and the n-hexane sorption was 69 mg/g.

**EXAMPLE 2 (Comparative)**

To 8.0 grams of the untreated catalyst (Ex. 1) was added 1.55 grams of dimethylphenylmethyl polysiloxane (Dow-550) dissolved in 40 cc of hexane. The catalyst was agitated in the silicone solution for several minutes and the hexane was distilled off by high vacuum distillation. After allowing the dry catalyst to cool to room temperature, the sample was then calcined in air at 1°C/min to 538°C and held for 3 hours. The silica modified catalyst had gained 3.7 wt.%, presumably as $SiO_2$.

The atmospheric TDP test was performed on the once-treated catalyst, as described in Example 1. The para-selectively of the catalyst was 67.3% at 4% toluene conversion, with a TDP rate constant of 226. The o-xylene diffusivity was decreased to $1.1 \times 10^{-6}$, and the n-hexane sorption was 68 mg/g.

**EXAMPLE 3**

To 5.75 grams of the once-treated catalyst (Ex. 2) was added 1.12 grams of dimethylphenylmethyl polysiloxane (Dow-550) dissolved in 40 cc of hexane. The catalyst was agitated in the silicone solution for several minutes and the hexane was distilled off by high vacuum distillation. After allowing the dry catalyst to cool to room temperature, the sample was then calcined in air at 1°C/min. to 538°C and held for 3 hours. The silica modified catalyst had gained an additional 5.0 wt.%, presumably as $SiO_2$.

The atmospheric TDP test was performed on the twice-treated catalyst, as described in Example 1. The para-selectivity of the catalyst was increased to 92.9% at 4% toluene conversion, with a TDP rate constant of 251. The o-xylene diffusivity was lowered to $0.29 \times 10^{-6}$, and the n-hexane sorption was 65 mg/g.

**EXAMPLE 4**

To 4.18 grams of the twice-treated catalyst (Ex. 3) was added 0.81 grams of dimethylphenylmethyl polysiloxane (Dow-550) dissolved in 40 cc of hexane. The catalyst was agitated in the silicone solution for several minutes and the hexane was distilled off by high vacuum distillation. After allowing the dry catalyst to cool to room temperature, the sample was then calcined in air at 1°C/min. to 538°C and held for 3 hours. The silica modified catalyst had gained an additional 0.8 wt.%, presumably as $SiO_2$.

The atmospheric TDP test was performed on the twice-treated catalyst, as described in Example 1. The para-selectivity of the catalyst was 99.1% at 4% toluene conversion, with a TDP rate constant of 249. The o-xylene diffusivity was further decreased to $0.073 \times 10^{-6}$, and the n-hexane sorption was 64 mg/g.

A comparison of the characteristics of the untreated catalyst and the three silicone treated catalysts described in Examples 1-4, is provided below in Table 1.

TABLE 1

| Characterization of Silicone Treated HZSM-5/$SiO_2$ Catalyst | | | | | |
|---|---|---|---|---|---|
| Catalyst | Silica Treatment | p-xyl/xyl $D/r^2$ | (%) | n-hexane sorption K | mg/g |
| Example 1 (Untreated) | None | $4.7 \times 10^{-6}$ | 37.0 | 167 | 69 |
| Example 2 | 1 | $1.1 \times 10^{-6}$ | 67.3 | 226 | 68 |
| Example 3 | 2 | $0.29 \times 10^{-6}$ | 92.9 | 251 | 65 |
| Example 4 | 3 | $0.073 \times 10^{-6}$ | 99.1 | 249 | 64 |

The results of Examples 1-4 show that multiple silicone coatings applied to a zeolite catalyst significantly change the characteristics of the catalyst. The triply-treated catalyst showed an extremely high para-selectivity of 99.1% compared to 37.0% for the untreated material. It will also be noted that the n-hexane sorption is similar for all of the Examples 1-4, indicating that the silica is deposited substantially exclusively at the crystal exterior. Finally, the triply-treated catalyst exhibits diffusivity about sixty-five times lower than that of the untreated material. This observation

corresponds, in a general way, with the estimated quantity of silica added to the catalyst, indicating that silica deposition has introduced a substantial diffusion barrier.

## EXAMPLE 5

Catalytic evaluation of selectivated catalyst was conducted in an automated unit with on-line sampling. One gram of the triply-coated material (Example 4) was loaded into a 0.25 inch diameter stainless steel tube reactor. The sample was heated to 538°C in 200 cc/min. air at a heating rate of 2.0°C/min. Pure toluene was then introduced at 485°C, 4 WHSV, 2 $H_2$/HC and 3550 kPa (500 psig). After 20 hours on stream the catalyst exhibited 80% p-xylene at 37% conversion. Varying the WHSV showed that very high p-xylene selectivities were possible, e.g., 96% p-xylene at 19% conversion with a WHSV of 16.

To determine the activity/selectivity of the selectivated catalyst, reactor temperature was varied to obtain a profile of toluene conversion as a function of temperature. For example, at 465°C, 4 WHSV, 2 $H_2$/HC and 3550 kPa (500 psig), the catalyst exhibited 93% p-xylene at 29% conversion.

## EXAMPLE 6

To determine the level of permanency of the selectivation, the used catalyst of Example 5 was air calcined. After 24 hours on stream, at 466°C, 4 WHSV, 2 $H_2$/HC and 3550 kPa (500 psig) on pure toluene feed, the catalyst profile had changed to 93% p-xylene at 30% conversion. Thus, excellent catalyst performance was maintained after the regeneration.

## EXAMPLE 7

In order to further examine the properties of the modified catalyst of Example 5, in situ trim-selectivation with 0.1 wt.% dimethylphenylmethyl polysiloxane (Dow-550) in toluene was initiated for a four hour period (485°C, 4 WHSV, 2$H_2$\HC and 3550 kPa). This trimming resulted in an increase in para-xylene selectivity, i.e., 91% para-xylene at 32% conversion versus 86% para-xylene at 35% conversion. Continued trimming under the same reaction conditions resulted in very high para-xylene selectivities.

## EXAMPLE 8

HZSM-5/$SiO_2$ with a crystal size of approximately 1.6 microns was subjected to four consecutive treatments with dimethylphenylmethyl polysiloxane (Dow-550) as described above and illustrated by Examples 2-4. A sample of this modified material weighing one gram was loaded into a 0.25 inch diameter, stainless steel tube reactor. The sample was pre-dried at 300°C for several hours. Then a selective toluene disproportionation reaction run was initiated using a pure toluene feed at 484°C, 4 WHSV, 2 $H_2$/HC and 3550 kPa (500 psig). The results are shown below in Table 2.

## EXAMPLE 9

Several grams of the multiply-coated catalyst described in Example 8 was steamed (100% steam) for four hours at 204°C, at 1 atmosphere. Then the activity and selectivity of the steamed catalyst were evaluated by performing a STDP run as described in Example 8. The results are shown in Table 2.

## EXAMPLE 10

Several grams of the multiply-coated catalyst described in Example 8 was steamed (100% steam) for four hours at 316°C, at 1 atmosphere. Then the activity and selectivity of the steamed catalyst were evaluated by performing a STDP run as described in Example 8. The results are shown in Table 2.

## EXAMPLE 11

Several grams of the multiply-coated catalyst described in Example 8 was steamed (100% steam) for four hours at 371°C, at 1 atmosphere. Then the activity and selectivity of the steamed catalyst were evaluated by performing a STDP run as described in Example 8. The results are shown in Table 2.

**EXAMPLE 12**

Several grams of the multiply-coated catalyst described in Example 8 was steamed (100% steam) for four hours at 500°C, at 1 atmosphere. Then the activity and selectivity of the steamed catalyst were evaluated by performing a STDP run as described in Example 8.

Table 2, below, provides data comparing activity and selectivity values for the unsteamed (Example 8) and variously steamed (Examples 9-12) multiply-coated catalysts.

TABLE 2

| Example | Catalyst | Para-selectivity at 25% toluene conversion | WHSV needed to achieve 25% toluene conversion |
|---|---|---|---|
| 8 | Unsteamed | 86 | 4 |
| 9 | Steamed at 204°C | 92 | 9 |
| 10 | Steamed at 316°C | 96 | 10 |
| 11 | Steamed at 371°C | 87 | 3.5 |
| 12 | Steamed at 500°C | 83 | 2 |

Catalyst samples steamed for four hours at 204°C (Ex. 9) or 316°C (Ex. 10) were both more active and more selective than unsteamed material, while higher temperature steaming (e.g., 500°C, 4 hours) resulted in significant loss in activity and a decrease in selectivity (Ex. 12). Material steamed at 371°C (Ex. 11) exhibited activity and selectivity commensurate with the unsteamed material (Ex. 8).

Importantly, the mildly steamed materials, Ex. 9 (204°C) and Ex. 10 (316°C) give 25% conversion at 9 and 10 WHSV, respectively, while the unsteamed material, Ex. 8, requires 4 WHSV. Thus, low temperature steaming gave a 2-3 fold increase in activity. The material steamed at high temperature, Ex. 12, required 2 WHSV for this conversion level, and thus, was only half as active as the unsteamed material.

**EXAMPLE 13**

The catalyst of this Example was prepared via a multiple coating procedure. 5.38 gm of untreated HZSM-5/$SiO_2$ material with a crystal size of 1.6 microns was subjected to three consecutive treatments with dimethylphenylmethyl polysiloxane (Dow-550) in dodecane. For each treatment ca. 1.9 gm of dimethylphenylmethyl polysiloxane (Dow-550) dissolved in 10 gm of dodecane served as the impregnation solution. After each treatment the catalyst was calcined in air at 5°C/min. to 538°C and held for 0.5 hr. The total weight gain of the catalyst was approximately 8 wt.%.

Toluene disproportionation was conducted in an automated unit with on-line sampling. Approximately one gram of catalyst extrudate was loaded into a 0.25 inch diameter, stainless steel tube reactor.

The catalytic run was initiated with pure toluene feed at 486°C, 4 WHSV, 2 $H_2$/HC and 3550 kPa (500 psig). Initially the catalyst exhibited 89% p-xylene at 30% conversion. After 20 hours on stream, catalyst activity improved slightly to 92% p-xylene at 28% conversion. At 8 WHSV, 96% p-xylene was obtained at 21% conversion. Thus, the change to a solvent of lower volatility appears not to have a detrimental effect on catalyst selectivity.

Varying the temperature over a range of ca. 80°C had little effect on results in that high para-selectivity values (>90%) were maintained. For example, 95% p-xylene was obtained at 23% conversion at 465°C.

**EXAMPLE 14**

Following the catalytic run described in Example 13, the catalyst was removed from the catalytic unit. To confirm the degree of permanency of the selectivation, the catalyst was regenerated by calcining rapidly in air at 5°C/min. to 538°C in a muffle furnace. After calcination, an initial sample, taken during a catalytic run at 486°C, 4 WHSV, 2 $H_2$/HC and 3550 kPa (500 psig) on pure toluene feed, showed 86% p-xylene at 24% conversion. After several hours on stream catalyst selectivity improved to 90% p-xylene at about 22% conversion. The overall loss in activity of the regenerated catalyst (ca. 25%) compared to the starting modified catalyst may be attributable to possible inadvertent steaming resulting from the rapid air calcination of the regeneration.

## EXAMPLE 15

To 20.0 grams of untreated HZSM-5/$SiO_2$ having a crystal size of 1.6 microns was added 3.88 grams of dimethylphenylmethyl polysiloxane (Dow-550) dissolved in 60 cc of hexane. The catalyst was agitated in the silicone solution for several minutes and the hexane was distilled off by high vacuum distillation. The dry catalyst was then calcined at 1°C/min. in nitrogen to 538°C. After allowing the sample to cool to room temperature, the sample was then calcined in air at 1°C/min. to 538°C and held for 3 hours. The silica-modified catalyst had gained 1.4 wt.%, presumably as $SiO_2$. The catalyst was then treated in a similar manner an additional three times with 6.77 grams, 6.82 grams and 6.78 grams of Dow-550, respectively. The resulting additional weight gains were 3.54 wt.%, 1.67 wt.% and 1.39 wt.%, respectively, for a total weight gain of about 8.23 wt.% after the four silicone treatments.

Catalytic activity and selectivity were assessed by performing a TDP run in an automated unit with on-line sampling. Approximately one gram of the modified catalyst was loaded into a 0.25 inch diameter, stainless steel tube reactor. The sample was heated to 538°C in 200 cc/min. air at a rate of 2.0°C/min. The catalytic run was initiated with pure toluene feed at 445°C, 4 WHSV, 2 $H_2$/HC and 3550 kPa (500 psig). A temperature scan showed that the catalyst was active and selective. For example, at 485°C, with other conditions identical, and at 22 hours on stream, the catalyst exhibited 88% p-xylene at 32% conversion. At 465°C, with other conditions unchanged, the catalyst showed 88% p-xylene at 26% conversion, after seven hours on stream.

Upon calcination to regenerate the catalyst, the sample produced 91% p-xylene and 30% conversion at 485°C, 4 WHSV, 2 $H_2$/HC and 3550 kPa (500 psig). Thus catalyst performance was maintained upon regeneration.

## EXAMPLE 16

To assess the effect of catalyst bulk handling, e.g., loading, unloading, etc., a sample of the extrudate of Example 15 was crushed to 14/30 mesh and tested for catalytic activity and selectivity. A one-gram sample was loaded and a catalytic run was performed as described in Example 15.

The catalytic run was initiated with pure toluene feed at 445°C, 4 WHSV, 2 $H_2$/HC and 3550 kPa (500 psig). A temperature scan showed that the catalyst was active and selective. After 8 hours on stream the catalyst exhibited 76% p-xylene at 37% conversion (485°C, 4 WHSV, 2 $H_2$/HC and 3550 kPa). After 19 hours on stream the catalyst exhibited 85% p-xylene at 32% conversion (485°C, 6 WHSV, 2 $H_2$/HC and 3550 kPa).

Thus, after an equivalent amount of time on stream, the crushed extrudate showed approximately the same activity/selectivity profile as the original modified material. These results suggest that physical damage to ex situ selectivated catalyst extrudate results in only minor losses (i.e., 3%) in catalyst selectivity; however, some of this loss may be regained with time on stream.

To assess the permanency of the selectivation and the effect thereon of the crushing process, the catalyst was regenerated by air calcination as described in Example 15. The catalyst showed 87% p-xylene at 32% conversion (485°C, 6 WHSV, 2 $H_2$/HC and 500 psig), roughly equivalent to the selectivity and activity of the crushed catalyst prior to regeneration. Crushing has apparently no effect on the regeneration behavior of the multiply-silicone coated catalyst.

## EXAMPLE 17

105.0 grams of untreated HZSM-5/$SiO_2$ (1/16 inch extrudate dried at 130°C) with a crystal size of 0.2 micron, was added to a solution of 10.0 grams dimethylphenylmethyl polysiloxane (Dow 550) dissolved in 92 grams of dodecane. The catalyst was mixed with the silicone solution at room temperature for two hours. Then the excess solvent was removed by filtration followed by a two step calcination procedure. The extrudate was heated in $N_2$ to 140°C and held for two hours then heated in $N_2$ at 2°C/min to 538°C and held for two hours. The sample was then cooled in $N_2$ to 300°C at which temperature air was introduced followed by heating at 2°C/min to 538°C and held for four hours. After cooling in $N_2$ to room temperature, the catalyst was determined to have gained 5.7 wt.%.

111.0 grams of the once-treated catalyst was added to a solution of 10.0 grams dimethylphenylmethyl polysiloxane (Dow 550) dissolved in 92 grams of dodecane. The catalyst was mixed with the silicone solution at room temperature for two hours. Then the excess solvent was removed by filtration followed by a two step calcination procedure. The extrudate was heated in $N_2$ to 140°C and held for two hours then heated in $N_2$ at 2°C/min to 538°C and held for two hours. The sample was then cooled in $N_2$ to 300°C at which temperature air was introduced followed by heating at 2°C/min to 538°C and held for four hours.

98.0 grams of the twice-treated catalyst was added to a solution of 9.6 grams dimethylphenylmethyl polysiloxane (Dow 550) dissolved in 88 grams of dodecane. The catalyst was mixed with the silicone solution at room temperature for two hours. Then the excess solvent was removed by filtration followed by a two step calcination procedure. The extrudate was heated in $N_2$ to 140°C and held for two hours then heated in $N_2$ at 2°C/min to 538°C and held for two hours. The sample was then cooled in $N_2$ to 300°C at which temperature air was introduced followed by heating

at 2°C/min to 538°C and held for four hours. After cooling in $N_2$ to room temperature, the catalyst was determined to have gained 2.0 wt.%.

96.0 grams of three times-treated catalyst was added to a solution of 9.4 grams dimethylphenylmethyl polysiloxane (Dow 550) dissolved in 86 grams of dodecane. The catalyst was mixed with the silicone solution at room temperature for two hours. Then the excess solvent was removed by filtration followed by a two step calcination procedure. The extrudate was heated in $N_2$ to 140°C and held for two hours then heated in $N_2$ at 2°C/min to 538°C and held for two hours. The sample was then cooled in $N_2$ to 300°C at which temperature air was introduced followed by heating at 2°C/min to 538° and held for four hours. After cooling in $N_2$ to room temperature, the catalyst was determined to have gained 2.0 wt.%.

Two grams of the four times-treated catalyst was evaluated in an automated unit with on line sampling. The sample was loaded into a 0.305" stainless steel tube reactor and then heated in hydrogen at 3.5°C/min to 425°C in 40 cc/min hydrogen. Pure toluene was introduced at 425°C at 4 WHSV, 1.5-2 $H_2$/HC and 2170 kPa (300 psig). Representative performance data are shown below in Table 3.

TABLE 3

| | | |
|---|---|---|
| Toluene Conversion (wt.%) | 30 | 30 |
| Para-Selectivity (wt.%) | 95 | 95 |
| Temperature (°C) | 428 | 426 |
| $H_2$/HC | 2.0 | 1.5 |
| Product Yields (wt.%) | | |
| Para-Xylene | 12.4 | 12.3 |
| Total Xylenes | 13.0 | 13.0 |
| Benzene | 14.4 | 14.8 |
| Bz/Xyl (molar) | 1.51 | 1.55 |
| Ethylbenzene | 0.4 | 0.5 |
| $C_5^-$ | 1.3 | 1.8 |
| $C_9^+$ | 0.4 | 0.4 |

**EXAMPLE 18**

50.0 grams of untreated HZSM-5/$SiO_2$ (1/16 inch extrudate, dried at 105°C) having a crystal size of 1.6 microns was added to a solution of 4.6 grams of dimethylphenylmethyl polysiloxane (Dow-550) dissolved in 49 grams of dodecane. The catalyst was allowed to contact/absorb the silicone solution for several minutes after which the dodecane was stripped under nitrogen at 210-220°C. The catalyst was then placed in a metal box and calcined in a muffle furnace at 2°C/min. in a 80%/20% $N_2$/air mixture (60 cc/min. total) to 538°C. The catalyst was held at 538°C for about 4 hours. After cooling under $N_2$ the silica modified catalyst was found to have gained 1.3 wt.%.

50.7 grams of the once-treated catalyst was added to a solution of 4.6 grams of dimethylphenylmethyl polysiloxane (Dow-550) dissolved in 50 grams of dodecane. The catalyst was allowed to contact/absorb the silicone solution for several minutes after which the dodecane was stripped under nitrogen at 210-220°C. The catalyst was then placed in a metal box and calcined in a muffle furnace at 2°C/min. in a 80%/20% $N_2$/air mixture (60 cc/min. total) to 538°C. The catalyst was held at 538°C for about 4 hours. After cooling under $N_2$ the silica modified catalyst was found to have gained 3.1 wt.%.

51.2 grams of the twice-treated catalyst was added to a solution of 2.4 grams of dimethylphenylmethyl polysiloxane (Dow-550) dissolved in 51 grams of dodecane. The catalyst was allowed to contact/absorb the silicone solution for several minutes after which the dodecane was stripped under nitrogen at 210-220°C. The catalyst was then placed in a metal box and calcined in a muffle furnace at 2°C/min. in a 80%/20% $N_2$/air mixture (60 cc/min. total) to 538°C. The catalyst was held at 538°C for about 4 hours. After cooling under $N_2$ the silica modified catalyst was found to have gained 2.1 wt.%.

The catalyst was divided into two 25 gm portions. One portion was steamed at 316°C for four hours in 100% steam, and was tested using the toluene disproportionation reaction as described below. The results are compared with the performance of the unsteamed portion of the multiple selectivated catalyst as well as with a coke selectivated catalyst.

TABLE 4

| Conditions: 3550 kPa, 4 WHSV, 2 $H_2$/HC | | | |
|---|---|---|---|
| | Coke Selectivation | Ex-Situ Steamed | Ex-Situ Unsteamed |
| Toluene Conversion (wt.%) | 30 | 30 | 29 |
| Para-Selectivity (wt.%) | 93 | 93 | 89 |
| Average Temperature (°C) | 464 | 471 | 474 |
| Product Yields (wt.%) | | | |
| Para-Xylene | 11.2 | 11.7 | 11.9 |
| Total Xylenes | 12.0 | 12.6 | 13.4 |
| Benzene | 14.8 | 14.4 | 12.7 |
| Bz/Xyl (molar) | 1.7 | 1.55 | 1.29 |
| Ethyl Benzene (EB) | 0.51 | 0.65 | 0.52 |
| EB/Total $C_8$ | 4.0 | 4.9 | 3.7 |
| $C_5^-$ | 2.4 | 2.0 | 2.0 |
| $C9^+$ | 0.57 | 0.64 | 0.64 |

The performance of the ex situ selectivated catalyst is apparently similar to that of the coke selectivated catalyst. For example, the triple silicone treated catalyst showed only slightly lower activity, as indicated by the higher average temperature required (471°C vs. 464°C). The yields of the ex situ selectivated catalyst, however, were generally more favorable than those of the coke selectivated sample. For example, the p-xylene yield was fractionally higher for the ex situ catalyst than for the coked catalyst.

## EXAMPLE 19

A comparison was made of the activity and selectivity of catalyst selectivated via a single silica coating and catalyst selectivated via the multiple coating technique in accordance with the present invention.

50.2 grams of untreated HZM-5/$SiO_2$ (1/16 inch extrudate, dried at 105°C) having a crystal size of 1.6 microns was added to a solution of 11.6 grams of dimethylphenylmethyl polysiloxane (Dow-550) dissolved in 50 grams of dodecane. The catalyst was allowed to contact/absorb the silicone solution for several minutes after which the dodecane was stripped under nitrogen at 210-220°C. The catalyst was then placed in a metal box and calcined in a muffle furnace at 2°C/minute in a 80%/20% $N_2$/air mixture (60 cc/min total) to 538°C. The catalyst was held at 538°C for ca. 4 hours. After cooling under $N_2$ the silica modified catalyst was found to have gained 5.2 wt.%.

The performance of the once-selectivated catalyst was evaluated using the toluene disprorortionation reaction as described below. The results are compared with the performance of the three times-selectivated catalyst of Example 16. It is shown that, at the same toluene conversion level, the triply treated catalyst is much more selective than the singly treated catalyst.

TABLE 5

| Single v. Multiple Ex-Situ Treatments | | |
|---|---|---|
| | Single | Triple |
| Attempted Wt.% Silica | 13.2 | 13.2 |
| Actual Wt.% Silica | 5.2 | 6.5 |
| Reaction Conditions | | |
| Temperature, °C | 445 | 465 |
| $H_2$/HC | 2 | 2 |
| Pressure (psig) | 500 | 500 |
| (kPa) | 3550 | 3550 |
| WHSV | 3 | 3 |

TABLE 5 (continued)

| Single v. Multiple Ex-Situ Treatments | | |
|---|---|---|
| | Single | Triple |
| Reaction Conditions | | |
| Toluene Conversion (Wt.%) | 29 | 29 |
| Xylene Yields (Wt.%) | | |
| Xylenes | 15.1 | 12.3 |
| Para Xylene | 4.9 | 11.3 |
| Para-Selectivity | 32 | 92 |

## EXAMPLE 20

The catalyst examined in this example was prepared via a multiple coating procedure. The untreated HZHM-5/$SiO_2$ material was subjected to two consecutive treatments with dimethyl-phenylmethyl polysiloxane (Dow-550), intended to add ca. 5 wt.% as silica. The detailed treatment is described below.

6.9 grams of untreated HZSM-5/$SiO_2$ with a crystal size of 1.6 microns (1/16 inch extrudate, dried at 300°C) was added to a solution of 0.65 grams of dimethylphenylmethyl polysiloxane (Dow-550) dissolved in 3 grams of dodecane. The catalyst was then placed in a vertical tube furnace and calcined at 2°C/minute in an 80%/20% $N_2$/air mixture (60 cc/min total) to 538°C. The catalyst was held at 538°C for ca. 3 hours. After cooling under $N_2$ the silica modified catalyst was found to have gained 2.9 wt.%.

7.1 grams of the modified catalyst was added to a solution of 0.66 grams of dimethylphenylmethyl polysiloxane (Dow-550) dissolved in 3 grams of dodecane. The catalyst was then placed in a vertical tube furnace and calcined at 2°C/minute in an 80%/20% $N_2$/air mixture (60 cc/min total) to 538°C. The catalyst was held at 538° for ca. 3 hours. After cooling under $N_2$ the silica modified catalyst was found to have gained 2.7 wt.%.

The performance of the twice modified catalyst was evaluated by performing a selective ethylbenzene disproportionation (SEBDP) run in an automated unit with on-line sampling. Approximately one gram of the modified catalyst was loaded into a 0.25 inch diameter, stainless steel tube reactor.

The catalytic run was initiated with ethylbenzene feed at 6 WHSV, 213 kPa (28 psig) and 0 $H_2$/HC. Data were obtained over a 20 day period on stream at temperatures between about 314°C and about 337°C. An aging rate of 0.6°C/day was observed after about 7 days. Over the entire run, p-diethylbenzene selectivity averaged about 97.4% at 13-14% ethylbenzene conversion.

## EXAMPLE 21

The performance of the modified catalyst of Example 20 was further evaluated by performing a SEBDP run using a sample of the modified catalyst in the presence of hydrogen diluent co-feed.

The catalytic run was initiated with ethylbenzene feed at 10 WHSV, 690-790 kPa (85-100 psig) and 1.0 $H_2$/HC. The activity and selectivity of the catalyst were monitored over a period of over 11 days at various temperatures.

After reaching an apparent steady state, the conversion of ethylbenzene remained at 20% for approximately 170 hours (1.0 $H_2$/HC, 350°C, 690 kPa, 10 WHSV). In the absence of hydrogen co-feed under these conditions, a catalyst aging rate of about 4°C/day is expected. In sharp contrast, in the presence of hydrogen little or no aging was observed over the time period studied.

## EXAMPLE 22 (Comparative)

Twenty grams of an n-propylamine silane polymer (Hydrosil 2627), was diluted with 20 grams deionized water. Seven grams of NaZSM-5/$SiO_2$ was added to this solution and allowed to equilibrate for 16 hours, after which the remaining water was evaporated at 130°C. The sample was then calcined in $N_2$ at 2°C/min to 538°C for two hours, followed by calcination in air at 538°C for two hours. 16.8% $SiO_2$ was found to have been added to the extrudate.

Catalytic evaluation of the selectivated catalyst was conducted at atmospheric pressure, 482°C and 4% toluene conversion. The p-xylene selectivity was 31% at these conditions.

**EXAMPLE 23**

Fifteen grams of NaZSM-5A/$SiO_2$ was added to 15 grams of an amino silane polymer/water solution and allowed to stand until dry. The catalyst was then heated in N2 at 2°C/min to 538°C then held for two hours. The sample was cooled to 300°C, air introduced and the sample heated at 2°C/min to 538°C and held for two hours. Based on the known silicon content of the n-propylamine silane polymer (Hydrosil 2627), 4.5% $SiO_2$ was found to have been added to the extrudate.
The once-modified catalyst was treated a second time using the procedure described for the first modification.
Thirteen grams of the twice-modified catalyst was added to 13 grams of a 27% amino silane polymer/water solution and allowed to stand until dry. The catalyst was then heated in $N_2$ at 2°C/min to 538°C then held for two hours. The sample was cooled to 300°C, air introduced and the sample heated at 2°C/min to 538°C and held for two hours.
Ten grams of the triply-treated catalyst was added to 10 grams of a 27% amino silane polymer/water solution and allowed to stand until dry. The catalyst was then heated in $N_2$ at 2°C/min to 538°C then held for two hours. The sample was ccoled to 300°C, air introduced and the sample heated at 2°C/min to 538°C and held for two hours.
After the fourth modification, the sample was exchanged with 1 M $NH_4NO_3$ at room temperature for one hour for a total of three exchanges.
Catalytic evaluation of the quadruply-treated catalyst (0.77 grams ash weight) was conducted in an automated unit with on line sampling. The sample was loaded into a 0.305" stainless steel tube reactor and then air calcined at 538°C for two hours to convert the ammonium form to the hydrogen form of ZSM-5. The sample was cooled to 300°C in nitrogen to remove excess oxygen. The sample was then heated in hydrogen at 3.5°C/min to 425°C in 40 cc/min hydrogen. Pure toluene was introduced at 425°C at 4 WHSV, 2 $H_2$/HC and 3550 kPa (500 psig). To determine the activity/selectivity performance of the selectivated catalyst, reactor temperature and toluene feed rate were varied to change toluene conversion. Representative toluene conversion, p-xylene selectivity and a product yield data are shown in Table 6.

TABLE 6

| Product Yields for Toluene Disproportionation | | | | |
|---|---|---|---|---|
| WHSV, $hr^{-1}$ | 4 | 8 | 12 | 16 |
| Products (wt.%) | | | | |
| $C_5^-$ | 2.0 | 0.9 | 0.5 | 0.3 |
| Benzene | 16.5 | 10.9 | 7.9 | 6.0 |
| Ethylbenzene | 0.5 | 0.3 | 0.1 | 0.1 |
| p-Xylene | 8.1 | 8.3 | 7.2 | 6.0 |
| Total xylenes | 16.2 | 12.4 | 9.4 | 7.4 |
| $C_9^+$ | 0.8 | 1.0 | 0.3 | 0.3 |
| Toluene Conversion, wt% | 35.9 | 24.9 | 18.3 | 14.1 |
| p-Xylene Selectivity, % | 50.3 | 67.2 | 75.8 | 80.7 |

80.7% p-xylene selectivity was observed at 14.1% toluene conversion for the quadruply-treated catalyst, compared to 31% p-xylene selectivity at 4% toluene conversion for the singly treated catalyst of Example 22.

**EXAMPLE 24**

A multiple selectivation procedure for a water based system is described below. The catalyst described in this example was selectivated in the sodium form using Hydrosil 2627, a water soluble n-propylamine silane polymer.
Ten grams of NaZSM-5/$SiO_2$ extrudate, with a crystal size of 0.20 micron, was added to 11 grams of a 16% solution of the n-propylamine silane polymer (Hydrosil 2627) in water and allowed to stand until dry. The catalyst was then heated in $N_2$ at 2°C/min. to 538°C then held for two hours. The sample was cooled to 300°C, air introduced and the sample heated at 2°C/min. to 538°C and held for two hours. Based on the silicon content of the silane polymer, 2.9 wt.% $SiO_2$ was added to the extrudate.
Ten grams of the once-modified catalyst was added to 11 grams of a 16% solution of the n-propylamine silane polymer (Hydrosil 2627) in water and allowed to stand until dry. The catalyst was then heated in $N_2$ at 2°C/min. to 538°C then held for 2 hours. The sample was cooled to 300°C, air introduced and the sample heated at 2°C/min. to

538°C and held for two hours. Based on the silicon content of the silane polymer, 2.9 wt.% $SiO_2$ was added to the extrudate, for 5.9 wt.% total added silica.

Two grams of the twice-modified catalyst was added to 2.5 grams of a 16% solution of the n-propylamine silane polymer (Hydrosil 2627) in water solution and allowed to stand until dry. The catalyst was then heated in $N_2$ at 2°C/min. to 538°C then held for two hours. The sample was cooled to 300°C, air introduced and the sample heated at 2°C/min. to 538°C and held for two hours. Based on the silicon content of the silane polymer, 3.3 wt.% $SiO_2$ was added to the extrudate, for 9.4 wt.% total added silica.

Two grams of the triply-modified catalyst was added to 2.5 grams of a 16% solution of the n-propylamine silane polymer (Hydrosil 2627) in water solution and allowed to stand until dry. The catalyst was then heated in $N_2$ at 2°C/min. to 538°C then held for two hours. The sample was cooled to 300°C, air introduced and the sample heated at 2°C/min. to 538°C and held for two hours. Based on the silicon content of the silane polymer, 3.3 wt.% $SiO_2$ was added to the extrudate, for 13.0 wt.% total added silica.

After the fourth modification, the sample was exchanged with 1M $NH_4NO_3$ at room temperature for one hour for a total of three exchanges.

Catalytic evaluation of the modified catalyst (0.783 grams ash weight) was conducted as described in Example 23. The modified catalyst exhibited 92% p-xylene and 14.2% conversion at 446°C, 16 WHSV, $2H_2$/HC, 3550 kPa (500 psig).

## EXAMPLE 25

Eleven grams of NaZSM-5/$SiO_2$, with a crystal size of 0.20 micron, was added to 11 grams of a 21% n-propylamine silane polymer (Hydrosil 2627)/water solution and allowed to stand until dry. The catalyst was then heated in $N_2$ at 2°C/min to 538°C then held for two hours. Based on the silicon content of the Hydrosil 2627, 3.6 wt.% $SiO_2$ was added to the extrudate.

The procedure described for the first modification was repeated.

Eight grams of the twice-treated catalyst was added to 8 grams of a 21% n-propylamine silane polymer (Hydrosil 2627)/ water solution and allowed to stand until dry. The catalyst was then heated in $N_2$ at 2°C/min to 538°C then held for two hours. The sample was cooled to 300°C, air was introduced and the sample was heated at 2°C/min to 538°C and held for two hours.

Six grams of the three times-treated catalyst was added to 6 grams of a 21% n-propylamine silane polymer (Hydrosil 2627)/water solution and allowed to stand until dry. The catalyst was then heated in $N_2$ at 2°C/min to 538°C then held for two hours. The sample was cooled to 300°C, air was introduced and the sample was heated at 2°C/min to 538°C and held for two hours.

After the fourth modification, the sample was exchanged with 1 M $NH_4NO_3$ at room temperature for one hour for a total of three exchanges. The sample was then air calcined at 538°C for two hours followed by steaming at 430°C for four hours with 100% $H_2O$(g).

The above catalyst was tested for ethylbenzene disproportionation at 6 WHSV, 310 kPa (30 psig), 315-330°C and 14% ethylbenzene conversion. The p-diethylbenzene selectivity was greater than 98% which is higher than that possible with conventional catalysts.

## EXAMPLE 26

Ten grams of MaZSM-5/$SiO_2$ was added to 10 grams of a 50% n-propylamine silane polymer (Hydrosil 2627) /water solution and allowed to stand until dry. The catalyst was then heated in $N_2$ at 2°C/min to 538° then held for two hours. The sample was cooled to 300°C, air was introduced, and then was heated at 2°C/min to 538°C and held for two hours.

9.5 grams of the once-modified catalyst was added to 9.5 grams of a 70% n-propylamine silane polymer (Hydrosil 2627)/ water solution and allowed to stand until dry. The catalyst was then heated in $N_2$ at 2°C/min to 538°C, then held for two hours. The sample was cooled to 300°C, air introduced and then heated at 2°C/min to 538°, and held for two hours. After the second modification, the sample was exchanged with 1 M $NH_4NO_3$ at room temperature for one hour for a total of three exchanges, then air calcined at 538°C for two hours.

Catalytic evaluation of the twice-modified catalyst in the disproportionation of toluene was conducted as described in Example 23. Representative selectivity/conversion data are shown in Table 7.

The sample was then coke trimmed to determine if higher p-xylene selectivity could be obtained. The reactor temperature was raised to 540°C, the feed rate to 6.5 WHSV, $H_2$/HC decreased to 0.5, nitrogen added at 3.5 $N_2$/HC and pressure decreased to 2860 kPa (400 psig). After 15 hours at these conditions, the reactor was set at 456°C, 4 WHSV, 2 $H_2$/HC and 3550 kPa (500 psig). Representative selectivity/conversion data are shown in Table 7. When the data before and after coke selectivation are compared, higher p-xylene selectivity is observed for the coke trimmed

catalyst at a given toluene conversion.

TABLE 7

| | Ex Situ Selectivated Catalyst | | Coke Trimmed Ex Situ Selectivated Catalyst | |
|---|---|---|---|---|
| Temperature °C | 447 | 447 | 455 | 455 |
| WHSV, $hr^{-1}$ | 7.9 | 15.8 | 3.9 | 3.9 |
| Products, wt% | | | | |
| $C_5^-$ | 0.5 | 0.2 | 0.9 | 1.5 |
| Benzene | 9.9 | 6.3 | 5.8 | 10.7 |
| Ethylbenzene | 0.2 | 0.1 | 0.1 | 0.4 |
| p-Xylene | 4.6 | 3.6 | 5.5 | 7.4 |
| Total xylenes | 12.4 | 7.9 | 7.4 | 12.2 |
| $C_9^+$ | 0.5 | 0.3 | 0.3 | 0.6 |
| Toluene Conversion, wt% | 23.5 | 14.8 | 14.6 | 25.4 |
| p-Xylene Selectivity, % | 37 | 46 | 74 | 61 |

**EXAMPLE 27**

A 70%/30% w/w surfactant mixture was prepared by mixing 14.0 grams of TRITON® X-100 and 6.0 grams of TRITON® X-305. 17.0 grams of the resultant surfactant mixture was then mixed with 983.0 grams of distilled water to produce a solution containing 1.7% surfactant in water. 60 grams of the water/surfactant solution was then mixed with 111.42 grams of DC-510 (Dow phenylmethyl silicone oil) and the mixture was emulsified in a blender for 1 minute to produce a 65% silicone oil/water emulsion. 6.5 grams of the 65% silicone oil emulsion was diluted to 65 grams with the water/surfactant solution and the diluted emulsion (6.5% oil/ water) was circulated in recycle mode over a packed bed containing 20.0 grams of 80/20 NaZSM-5/$SiO_2$ for a total of 12 hours in 3 successive treatments with air calcinations to 538°C between each impregnation. The final catalyst was $NH_4^+$ exchanged 3 times for 1 hour each with 1M $NH_4NO_3$ at room temperature. $SiO_2$ addition to the final catalyst measured by increase in weight was 6%.

The selectivated catalyst was used to convert toluene at 446°C, 2170 kPa (300 psig), and a hydrogen/hydrocarbon mole ratio of 2. The results are indicated in Table 8 and show a para-xylene selectivity of 55.4% at 29.6% toluene conversion.

A 4th aqueous silicone emulsion/air calcination sequence was effected on the selectivated catalyst and an additional 1% $SiO_2$ was added, as measured by the increase in catalyst weight after calcination. The resultant catalyst was used to convert toluene at 425°C, 2170 kPa (300 psig), and a hydrogen/hydrocarbon mole ratio of 2. Results in Table 8 show a para-xylene selectivity of 88.4% at 30.9% toluene conversion.

Untreated 80/20 HZSM-5/$SiO_2$ catalyst with a crystal size of 0.2 micron was used to convert toluene at 446°C, 2170 kPa (300 psig), and a hydrogen/hydrocarbon mole ratio of 2. Results in Table 8 show a para-xylene selectivity of 27.2% at 61.6% toluene conversion.

TABLE 8

| Toluene Disproportionation Results | | | |
|---|---|---|---|
| Example # | 15 | 16 | 17 |
| Temperature, °C | 446 | 446 | 425 |
| $H_2$:HC (mole:mole) | 2 | 2 | 2 |
| WHSV, $hr^{-1}$ | 10 | 4 | 6 |
| % Toluene Conversion | 61.6 | 29.6 | 30.9 |
| p-xylene selectivity | 27.2 | 55.4 | 88.4 |

**EXAMPLE 28**

Silica modified HZSM-5 was prepared by a 2-step aqueous silicone emulsion impregnation procedure. To 1.13 grams phenylmethyl silicone emulsion (65% oil/0.6% surfactant) and 7.20 grams distilled water was added 4.50

grams HZSM-5 with a crystal size of 0.2 micron. Water was distilled using a rotovap. The product was program calcined in air at 1°C/minute to 538°C, then 6 hours at 538°C. The calcined catalyst weighed 4.89 g (7.98% added silica).

The above procedure was repeated using 1.23 grams phenyl-methyl silicone emulsion, 3.30 grams distilled water, and 2.46 grams of the silica modified HZSM-5. After water distillation and air calcination, the catalyst weighed 2.80 grams, corresponding to 19.6% added silica.

Coke trim-selectivation of this silica modified HZSM-5 catalyst (2.00 grams) was carried out at 579°C, 790 kPa (100 psig), and 0.5 WHSV using toluene feed. A mixture of nitrogen and hydrogen ($N_2/H_2 = 8$) was passed through the catalyst at 19.9 cc/minute. After 72 hours, the selectivation was terminated. The following table (Table 9) shows toluene conversion and p-xylene selectivity during trim-selectivation:

TABLE 9

| Time on Stream, Hrs. | Toluene Conversion. Wt.% | p-Xylene in Xylenes, Wt.% |
|---|---|---|
| 8 | 51 | 28 |
| 18 | 45 | 42 |
| 24 | 43 | 52 |
| 46 | 32 | 78 |
| 70 | 25 | 89 |

After trim-selectivation, the catalyst was tested under process conditions of 465°C, 3550 kPa (500 psig), 3 WHSV, and a hydrogen/hydrocarbon ratio = 2.0. At 23% toluene conversion, the p-xylene selectivity was a high 95.4%. The temperature was increased to 485°C. The toluene conversion increased to 28% with p-xylene selectivity still high at 93.2%.

## Claims

1. A process for modifying the shape selectivity of an aluminosilicate zeolite catalyst having a silica to alumina molar ratio less than 500 and comprising exposing the catalyst to at least two ex situ selectivation sequences, wherein each ex situ selectivation sequence includes the steps of contacting the catalyst with a selectivating agent in a carrier and subsequently calcining the catalyst, **characterised in that** the selectivating agent is an organosilicon compound.

2. The process of claim 1, wherein the catalytic molecular sieve has been modified by between two and six ex situ selectivation sequences.

3. The process of claim 1 or claim 2, wherein the selectivating agent is selected from silicones, silicone polymers, silanes, alkoxysilanes and aminosilanes.

4. The process of claim 3, wherein the selectivating agent is selected from silicone polymers having repeating units **characterised by** the formula:

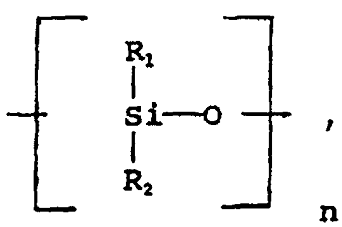

wherein $R_1$ and $R_2$ are independently selected from the group consisting of hydrogen, halogen, hydroxyl, alkyl, alkoxy, halogenated alkyl, aryl, halogenated aryl, aralkyl, halogenated aralkyl, alkaryl, and halogenated alkaryl, and n is between 2 and 1000; and organosilanes obeying the formula:

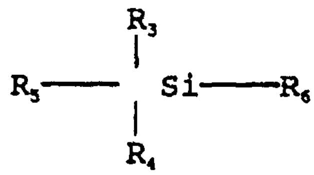

,

wherein $R_3$, $R_4$, $R_5$ and $R_6$ are independently selected from the group consisting of hydrogen, halogen, hydroxyl, alkyl, alkoxy, aminoalkyl, halogenated alkyl, aryl, halogenated aryl, aralkyl, halogenated aralkyl, alkaryl, and halogenated alkaryl.

**5.** The process of any preceding claim, wherein the carrier is an organic phase.

**6.** The process of claim 5, wherein the organic carrier comprises a linear, branched, or cyclic hydrocarbon.

**7.** The process of claim 5 or claim 6, wherein the organic carrier is a paraffin containing at least 7 carbon atoms.

**8.** The process of any one claims claim 1 to 4, wherein the carrier includes an aqueous phase.

**9.** The process of claim 8, wherein the aqueous phase comprises an emulsion including the selectivating agent and a surfactant.

**10.** The process of any preceding claim, wherein the zeolite has a silica to alumina molar ratio of 12 to 100.

**11.** The process of any preceding claim, wherein the zeolite has a silica to alumina molar ratio of 20 to 60.

**12.** The process of any preceding claim, wherein each calcination step is effected at a temperature of at least 250°C.

**13.** The process of any preceding claim, further comprising the step of steaming the modified catalytic molecular sieve under conditions comprising from 1% to 100% water vapor, a temperature of 100°C to 600°C, a pressure of 0.07 to 350 kPa (0.01 to 50 psia), for a time of from 0.1 to 12 hours.

**14.** The process of any preceding claim, further comprising the step of in situ trim-selectivating the modified catalytic molecular sieve.

**15.** The process of claim 14, wherein the in situ trim-selectivation comprises contacting the modified catalytic molecular sieve with a thermally decomposable organic compound selected from paraffins, cycloparaffins, olefins, cycloolefins, aromatics, alcohols, aldehydes, ethers, ketones, phenols, heterocyclics, and mixtures thereof, at a temperature in excess of the decomposition temperature of the thermally decomposable organic compound.

**16.** The process of claim 14, wherein the in situ trim-selectivating step comprises contacting the modified catalytic molecular sieve with a reaction stream comprising an alkyl-substituted benzene and an organosilicon trim-selectivating agent, at reaction conditions for shape selective disproportionation of the alkyl-substituted benzene.

**17.** The process of any preceding claim wherein the zeolite has a Constraint Index of 1 to 12.

**18.** The process of any preceding claim wherein the zeolite is ZSM-5.

**19.** Use of a zeolite catalyst modified by a process as claimed in any preceding claim in a para-selective alkylaromatic conversion process.

**20.** The use of claim 19 wherein the conversion process is the selective disproportionation of an alkylbenzene to a para-diakylbenzene.

**21.** The use of claim 20 wherein the alkylbenzene is toluene or ethyibenzene.

**Patentansprüche**

1. Verfahren zum Modifizieren der Formselektivität eines Aluminosilicat-Zeolithkatalysators mit einem Siliciumdioxid/Aluminiumoxid-Molverhältnis von weniger als 500, bei dem der Katalysator mindestens 2 ex situ durchgeführten Selektivierungssequenzen ausgesetzt wird, wobei jede ex situ durchgeführte Selektivierungssequenz die Schritt des Kontaktes des Katalysators mit einem Selektivierungsmittel in einem Träger und des anschließenden Kalzinierens des Katalysators umfaßt, **dadurch gekennzeichnet, daß** das Selektivierungsmittel eine organische Siliciumverbindung ist.

2. Verfahren nach Anspruch 1, wobei das katalytische Molekularsieb durch 2 bis 6 ex situ durchgeführte Selektivierungssequenzen modifiziert worden ist.

3. Verfahren nach Anspruch 1 oder Anspruch 2, wobei das Selektivierungsmittel aus Siliconen, Siliconpolymeren, Silanen, Alkoxysilanen und Aminosilanen ausgewählt ist.

4. Verfahren nach Anspruch 3, wobei das Selektivierungsmittel aus Siliconpolymeren mit sich wiederholenden Einheiten, die mit der Formel

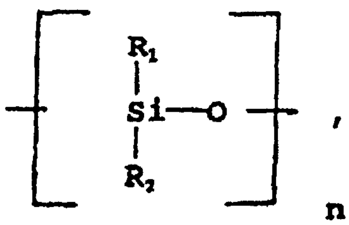

**gekennzeichnet** sind, worin $R_1$ und $R_2$ unabhängig aus der Gruppe ausgewählt sind, die aus Wasserstoff, Halogen, Hydroxyl, Alkyl, Alkoxy, halogeniertem Alkyl, Aryl, halogeniertem Aryl, Aralkyl, halogeniertem Aralkyl, Alkaryl und halogeniertem Alkaryl besteht, und n zwischen 2 und 1000 liegt, und Organosilanen ausgewählt ist, die der Formel entsprechen:

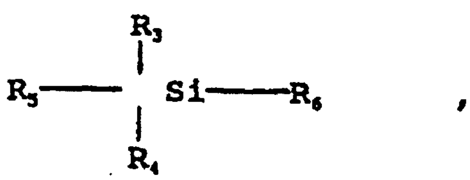

worin $R_3$, $R_4$, $R_5$ und $R_6$ unabhängig aus der Gruppe ausgewählt sind, die aus Wasserstoff, einem Halogen, Hydroxyl, Alkyl, Alkoxy, Aminoalkyl, halogeniertem Alkyl, Aryl, halogeniertem Aryl, Aralkyl, halogeniertem Aralkyl, Alkaryl und halogeniertem Alkaryl besteht.

5. Verfahren nach einem der vorstehenden Ansprüche, wobei der Träger eine organische Phase ist.

6. Verfahren nach Anspruch 5, wobei der organische Träger einen linearen, verzweigten oder cyclischen Kohlenwasserstoff umfaßt.

7. Verfahren nach Anspruch 5 oder Anspruch 6, wobei der organische Träger ein Paraffin mit mindestens 7 Kohlenstoffatomen ist.

8. Verfahren nach einem der Ansprüche 1 bis 4, wobei der Träger eine wäßrige Phase einschließt.

9. Verfahren nach Anspruch 8, wobei die wäßrige Phase eine Emulsion umfaßt, die das Selektivierungsmittel und ein oberflächenaktives Mittel einschließt.

10. Verfahren nach einem der vorstehenden Ansprüche, wobei der Zeolith ein Siliciumdioxid/Aluminiumoxid-Molverhältnis von 12 bis 100 hat.

11. Verfahren nach einem der vorstehenden Ansprüche, wobei der Zeolith ein Siliciumdioxid/Aluminiumoxid-Molverhältnis von 20 bis 60 hat.

12. Verfahren nach einem der vorstehenden Ansprüche, wobei jeder Kalzinierungsschritt bei einer Temperatur von mindestens 250°C durchgeführt wird.

13. Verfahren nach einem der vorstehenden Ansprüche, das ferner einen Schritt umfaßt, bei dem das modifizierte katalytische Molekularsieb während eines Zeitraums von 0,1 bis 12 Stunden bei Bedingungen einer Dampfbehandlung unterzogen wird, die 1 bis 100 % Wasserdampf, eine Temperatur von 100 bis 600°C, einen Druck von 0,07 bis 350 kPa (0,01 bis 50 psia) umfassen.

14. Verfahren nach einem der vorstehenden Ansprüche, das ferner den Schritt der in situ durchgeführten Trimmselektivierung des modifizierten katalytischen Molekularsiebs umfaßt.

15. Verfahren nach Anspruch 14, wobei die in situ durchgeführte Trimmselektivierung den Kontakt des modifizierten katalytischen Molekularsiebs mit einer thermisch zersetzbaren organischen Verbindung, die aus Paraffinen, Cycloparaffinen, Olefinen, Cycloolefinen, Aromaten, Alkoholen, Aldehyden, Ethern, Ketonen, Phenolen, heterocyclischen Verbindungen und Gemischen davon ausgewählt ist, bei einerTemperatur umfaßt, die über der Zersetzungstemperatur der thermisch zersetzbaren organischen Verbindung liegt.

16. Verfahren nach Anspruch 14, wobei der Schritt der in situ durchgeführten Trimmselektivierung in Kontakt des modifizierten katalytischen Molekularsiebs mit einem Reaktionsstrom, der ein alkylsubstituiertes Benzol und ein Trimmselektivierungsmittel aus einer organischen Siliciumverbindung umfaßt, bei Reaktionsbedingungen für die formselektive Disproportionierung des alkylsubstituierten Benzols umfaßt.

17. Verfahren nach einem der vorstehenden Ansprüche, wobei der Zeolith einen Zwangsindex von 1 bis 12 hat.

18. Verfahren nach einem der vorstehenden Ansprüche, wobei der Zeolith ZSM-5 ist.

19. Verwendung eines Zeolithkatalysators, der nach einem Verfahren nach einem der vorstehenden Ansprüche modifiziert worden ist, bei einem paraselektiven Umwandlungsverfahren von Alkylaromaten.

20. Verwendung nach Anspruch 19, wobei das Umwandlungsverfahren die selektive Disproportionierung von Alkylbenzol in p-Dialkylbenzol ist.

21. Verwendung nach Anspruch 20, wobei das Alkylbenzol Toluol oder Ethylbenzol ist.

**Revendications**

1. Procédé pour modifier la sélectivité de forme d'un catalyseur à base de zéolite de type aluminosilicate ayant un rapport molaire de la silice à l'alumine inférieur à 500 et comprenant l'exposition du catalyseur à au moins deux séquences de traitement *ex situ* contribuant à la sélectivité, dans lequel chaque séquence de traitement *ex situ* contribuant à la sélectivité comprend les étapes de mise en contact du catalyseur avec un agent contribuant à la sélectivité dans un support et ensuite de calcination du catalyseur, **caractérisé en ce que** l'agent contribuant à la sélectivité est un composé organosilicique.

2. Procédé suivant la revendication 1 dans lequel le tamis moléculaire catalytique a été modifié par un nombre de deux à six séquences de traitement *ex situ* contribuant à la sélectivité.

3. Procédé suivant la revendication 1 ou la revendication 2, dans lequel l'agent contribuant à la sélectivité est choisi parmi des silicones, des polymères de silicone, des silanes, des alcoxysilanes et des aminosilanes.

4. Procédé suivant la revendication 3 dans lequel l'agent contribuant à la sélectivité est choisi parmi des polymères de silicone formé d'unités répétitives **caractérisées par** la formule:

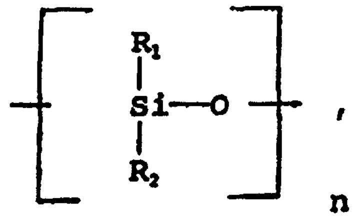

dans laquelle $R_1$ et $R_2$ sont choisis, indépendamment l'un de l'autre, dans le groupe consistant en atome d'hydrogène, atome d'halogène, groupe hydroxy, alkyle, haloalkyle, aryle, haloaryle, aralkyle, haloaralkyle, alcaryle et haloalcaryle, et n est compris entre 2 et 1000; et des organosilanes correspondant à la formule:

dans laquelle $R_3$, $R_4$, $R_5$ et $R_6$ sont, indépendamment les uns des autres, choisis dans le groupe consistant en atome d'hydrogène, atome d'halogène, groupe hydroxy, groupe alkyle, alcoxy, alkylamino, haloalkyle, aryle, haloaryle, aralkyle, haloaralkyle, alcaryle et haloalcaryle.

**5.** Procédé suivant l'une quelconque des revendications précédentes, dans lequel le support est une phase organique.

**6.** Procédé suivant la revendication 5, dans lequel le support organique comprend un hydrocarbure linéaire, ramifié ou cyclique.

**7.** Procédé suivant la revendication 5 ou la revendication 6, dans lequel le support organique est une paraffine contenant au moins 7 atomes de carbone.

**8.** Procédé suivant l'une quelconque des revendications 1 à 4, dans lequel le support comprend une phase aqueuse.

**9.** Procédé suivant la revendication 8, dans lequel la phase aqueuse est constituée d'une émulsion comprenant l'agent contribuant à la sélectivité et un tensioactif.

**10.** Procédé suivant l'une quelconque des revendications précédentes, dans lequel la zéolite a un rapport molaire compris entre silice et alumine de 12 à 100.

**11.** Procédé suivant l'une quelconque des revendications précédentes, dans lequel la zéolite a un rapport molaire compris entre silice et alumine de 20 à 60.

**12.** Procédé suivant l'une quelconque des revendications précédentes, dans lequel chaque étape de calcination est effectuée à une température d'au moins 250°C.

**13.** Procédé suivant l'une quelconque des revendications précédentes comprenant de plus l'étape de traitement à la vapeur du tamis moléculaire catalytique modifié dans des conditions comprenant de 1% à 100% de vapeur d'eau, une température de 100°C à 600°C, une pression de 0,07 à 350 kPa (0,01 à 50 psia), pendant une durée de 0,1 à 12 heures.

**14.** Procédé suivant l'une quelconque des revendications précédentes comprenant de plus l'étape de traitement *in situ* ajustant la sélectivité du tamis moléculaire catalytique modifié.

**15.** Procédé suivant la revendication 14, dans lequel le traitement *in situ* ajustant la sélectivité comprend la mise en contact du tamis moléculaire catalytique modifié avec un composé organique décomposable thermiquement choisi parmi les paraffines, les cycloparaffines, les oléfines, les cyclooléfines, les produits aromatiques, les alcools, les aldéhydes, les éthers, les cétones, les phénols, les composés hétérocycliques, et leurs mélanges, à une température supérieure à la température de décomposition du composé organique décomposable thermiquement.

**16.** Procédé suivant la revendication 14, dans lequel l'étape de traitement *in situ* ajustant la sélectivité comprend la mise en contact du tamis moléculaire catalytique modifié avec un courant de réaction comprenant un benzène à substituant alkyle et un agent organosilicique ajustant la sélectivité, dans des conditions de réaction de disproportionation sélective quant à la forme du benzène à substituant alkyle.

**17.** Procédé suivant l'une quelconque des revendications précédentes, dans lequel la zéolite a un Indice de Contrainte de 1 à 12.

**18.** Procédé suivant l'une quelconque des revendications précédentes, dans lequel la zéolite est une ZSM-5.

**19.** Utilisation d'un catalyseur à base de zéolite modifié par un procédé suivant l'une quelconque des revendications précédentes dans un procédé de conversion para-sélective d'alkylaromatiques.

**20.** Utilisation suivant la revendication 19, dans laquelle le procédé de conversion est la disproportionation sélective d'un alkylbenzène en paradiakylbenzène.

**21.** Utilisation suivant la revendication 20, dans laquelle l'alkylbenzène est le toluène ou l'éthylbenzène.